# FASCICULE DE BREVET EUROPEEN

(11) **EP 0 315 552 B1**
(45) Date de publication et mention de la délivrance du brevet: **14.12.1994**
(21) Numéro de dépôt: 88420341.5
(22) Date de dépôt: 07.10.1988
(51) Int. Cl.: A61K 35/54, A61K 9/20

(54) **Nouvelles formes galéniques d'oeufs de caille pour administration par voie per- et sublinguale dans le traitement des maladies allergiques et leur procédé de préparation**
Galenische Formen von Wachteleiern für die per- und sublinguale Verabreichung zur Behandlung von Allergien und ihr Herstellungsverfahren
Galenical forms of quail eggs for per- and sublingual administration in the treatment of allergic diseases, and their preparation process

(30) Priorité: 08.10.1987 FR 8714475; 04.12.1987 FR 8717252
(43) Date de publication de la demande: 10.05.1989
(73) Titulaire: MEDIBREVEX SA, 74940 Annecy Le Vieux (FR)
(72) Inventeur: Clairet, épouse Ayache, Josiane, F-38000 Grenoble (FR); Ayache, Jean-Jacques, F-38000 Grenoble (FR); Bruttmann, Georges, F-38000 Grenoble (FR); Pedrali, Patrick, F-74000 Annecy (FR); Robert, Serge, B-1440 Braine le Château (BE)
(74) Mandataire: Maureau, Philippe

(56) Documents cités:
- EP-A- 0 032 344
- FR-A- 2 356 426
- FR-A- 2 477 017
- FR-A- 2 538 248
- FR-A- 2 568 126
- M. Traisnel et al., GALENICA 16 MEDICAMENTS HOMEOPATHIQUES, "Notions pratiques de pharmacie homéopathique", éd. 2, 1986, Technique et Documentation, Paris (FR); pp. 77-99#

## Description

La présente invention concerne de nouvelles formes galéniques d'oeufs de caille pour administration par voie per- et sublinguale dans le traitement des maladies allergiques et leur procédé de préparation.

Depuis 1967, un certain nombre d'observations cliniques faites chez des éleveurs de cailles atteints de maladies allergiques (asthme) ont conduit à penser que les améliorations cliniques constatées dans de nombreux cas provenaient de l'absorption d'oeufs de caille.

En 1968, une étude fut proposée pour traiter des cas rebelles à toute thérapeutique : en 1969, un essai de traitement fut également conduit dans le rhume des foins. Puis tous les équivalents allergiques furent traités : rhinites, toux spasmodiques, asthmes, conjonctivites allergiques, eczémas, etc...

De 1968 à 1978, 800 malades environ furent traités par les oeufs de caille, dans un essai multicentrique comportant des Allergologues, des Pédiatres, et des Généralistes. Ces résultats furent colligés par le Docteur J.C. TRUFFIER dans la revue "LA CLINIQUE", n° 22, 15 Mars 1978, 2-4.

Le schéma thérapeutique proposé par J.C. TRUFFIER est le suivant :
absorption en moyenne de 6 oeufs, de caille par jour en une seule prise pendant 9 jours, arrêt 9 jours, reprise 9 jours avec parfois une cure de consolidation de 6 jours, toujours après 9 jours d'arrêt.

On a pu constater, avec ce traitement à base d'oeufs entiers, des améliorations sensibles dans 60 % à 80 % des cas.

Cette prescription fut également étendue aux enfants entre 5 et 10 ans : les améliorations observées furent alors de l'ordre de 88 % des cas.

Cependant, certains malades reportèrent les incidents suivants :
- prise de poids (23 %),
- hypercholestérolémie (2 %),
- troubles digestifs (16 %),
- dégout au moment de la prise d'oeufs (22 %).

De nombreux malades, satisfaits des résultats cliniques qu'ils avaient obtenus, regrettèrent vivement de ne pas pouvoir disposer d'une forme d'oeuf de caille plus agréable et plus simple d'emploi, donnant des résultats cliniques identiques.

On essaya de mettre ces oeufs sous forme de poudres et de lyophilisats, mais on dut constater alors que les résultats cliniques étaient médiocres.

On peut penser que I'inefficacité de ces formes galéniques était probablement due au choc thermique infligé au blanc d'oeuf.

Les inventeurs se sont alors efforcés d'analyser la nature du phénomène. Ils ont pu déterminer ce qui suit :
- L'analyse des fragments obtenus par le bromure de cyanogène et par la protéase staphylococcique (KURISAKI) montre que l'activité allergénique se situe au niveau d'un peptide commun à plusieurs fragments d'une chaîne polypeptidique.
- L'ovomucoïde est partiellement dénaturée par une augmentation de température au-delà de 38° à 40°C.
- L'ovomucoïde est violemment histamino-libératrice.
- Le jaune d'oeuf (albumine, lipides comportant des vitamines A et B, des sels minéraux et des pigments caroténoïdes ainsi que la lutéine) peut être allergénique.
- Les antigen'es de l'oeuf ont une activité puissante (à la dose de 20 à 45 µg/ml, les polypeptides peuvent créer des réactions d'allergie chez un sujet sensibilisé).

Les inventeurs ont alors eu l'idée d'appliquer la loi d'HAHNEMANN afin de supprimer les effets secondaires d'une thérapeutique à base d'oeuf entier et d'en conserver les éléments positifs.

Et conformément à l'invention, une composition médicamenteuse comprend un support divisé adapté à un délitement per et sublingual du principe actif, et une partie aliquote d'oeufs de caille (coturnix ovum) obtenue par dilution hahnemanienne d'une solution mère dans l'eau contenant un mélange en parties pondérales égales de jaunes et de blancs d'oeufs, dont la concentration dosable est au plus égale à 3DH (troisième dilution hahnemanienne), et contenue sur ledit support.

Cette voie per- et sublinguale est utilisée a cause de sa configuration anatomique qui en fait une entité par rapport aux autres éléments de la cavité buccale.

Schématiquement, on peut décrire la région sublinguale de la manière suivante (chacun des éléments constitutifs ayant des propriétés utiles dans l'administration des médicaments introduits par cette voie).

Globalement, la région sublinguale comprend :
- la face inférieure de la langue qui sert de plafond, très riche en vaisseaux sanguins : veines, artères et vaisseaux lymphatiques ;
- le plancher de la bouche qui constitue la partie inférieure de la région sublinguale. C'est également une région anatomique qui comporte beaucoup de vaisseaux sanguins et surtout un très riche réseau veineux, des artères et des vaisseaux lymphatiques ;
- les bords de la région sublinguale sont formés par les parties montantes du maxillaire inférieur, les gencives et les dents.

Dans le plancher de la bouche, on trouve des glandes salivaires, les glandes sublinguales, qui secrètent de la salive dès qu'il existe un contact avec la région sublinguale. Par sa composition, la salive participe activement au délitement du produit pharmacologiquement actif présenté sous cette forme galénique sublinguale particulière selon l'invention.

Par ailleurs, la structure histologique montre l'existence de cellules immuno-compétentes en grand nombre qui vont, dans certains cas, favoriser l'activité médicamenteuse.

Par rapport aux autres constituants de la bouche : intérieur des joues, partie supérieure de la langue, la région sublinguale apparaît donc comme une entité anatomique particulière qui en fait une voie d'administration médicamenteuse privilégiée comparable à l'administration des médicaments par voie injectable.

Tous les autres médicaments dont le délitement se fait dans la bouche sont forcément avalés.

Dans la forme galénique sublinguale selon l'invention, l'activité du produit est fondée sur sa possibilité d'absorption rapide sans déglutition. L'avantage que présente cette forme galénique par rapport aux autres formes galéniques utilisées à l'intérieur de la cavité buccale est que le produit passe directement dans le sang, ce qui évite le métabolisme hépatique ; le produit agit donc plus rapidement avec une efficacité maximale.

Le mode de préparation de ces formes galéniques met en jeu des techniques homéopathiques et l'invention concerne les applications homéopatiques de ces formes galéniques. Les Inventeurs ont pu, par ailleurs, déterminer que les premières dilutions hahnemaniennes, jusqu'à la troisième dilution hahnemanienne incluse, renfermaient encore des quantités dosables du principe actif, ce qui rend donc possible d'appliquer également les formes galéniques selon l'invention en allopathie.

Avantageusement, les nouvelles formes galéniques (coturnix ovum) sont constituées d'extraits d'oeufs de caille (souche B mina) réalisées en troisième dilution hahnemanienne et dans toutes les autres dilutions à partir d'une solution mère, obtenue par trituration contenant un mélange de jaunes et de blancs d'oeufs.

Le procédé de préparation de ces formes galéniques va maintenant être décrit en détail.

On part d'oeufs entiers de taille moyenne, de couleur gris-beige, avec des taches grises plus foncées ou brunes.
Poids moyen : environ 13,6 g. (12 g. à 15,5 g.).
Dimensions : hauteur environ 35 mm., Diamètre environ 27 mm., volume moyen environ 10 ml.

### Réactions d'identité.

1° - Blanc d'oeuf : on prépare une solution à 10 pour cent (V/V) dans l'eau distillée (solution A).
   a) - 1 ml de la solution A est additionné de 1 ml de solution d'acide trichloracétique R. Il se produit rapidement un trouble blanchâtre.
   b) - 1 ml de la solution A est additionné de 1 ml de ninhydrine R. On chauffe au bain-marie pendant quelques minutes. Il apparaît une coloration violacée.
   c) - La solution mousse fortement par agitation.
2° - Jaune d'oeuf : on prépare une solution à 10 %, (V/V) dans l'eau distillée (solution B).
   a) - La solution mousse par agitation.
   b) - A 5 ml de solution B, on ajoute 5 ml de chloroforme R. On agite avec précaution pour éviter les émulsions possibles. On laisse reposer. La solution chloroformique est colorée en jaune.

On prélève 2 ml de solution chloroformique par filtration sur un filtre hydrophobe.

On ajoute 1 ml d'anhydride acétique R. On mélange soigneusement. On ajoute une goutte d'acide sulfurique concentré R.

Il se forme progressivement une suite de colorations rose, violette, bleue et finalement verte.

On prépare ensuite les dilutions homéopathiques, par exemple en troisième dilution hahnemanienne (3e DH), à partir d'un mélange du blanc et du jaune homogénisé par battage.

Le produit fini est mis en gélules transparentes contenant soit des globules, soit une trituration de la dilution homéopathique correspondante. Les triturations se font au moyen de lactose (Ph. Eur.) et de saccharose (Ph. Eur.) dans les proportions respectives de 12 à 88 (P/P).
- Le produit ne peut être analysé étant donné qu'il s'agit de dilutions homéopathiques.
- La durée de validité est fixée à 5 ans en concordance avec toutes les préparations homéopathiques.

Le produit terminé, des contrôles de stérilité sont réalisés :
- sur les granules à l'air libre.
- sur les granules conservés sous blisters au laboratoire.
- sur les granules conservés sous blisters, après expédition et stockage de deux mois dans des conditions normales.

Les nouvelles formes galéniques d'oeufs de caille selon l'invention sont indiquées pour toutes les maladies allergiques.

Elles ne présentent aucune contre-indication.

Leur mode d'administration se fait par voie sublinguale stricte à raison d'une gélule par jour en moyenne.

Pour l'usage pédiatrique, le contenu de la gélule est tout simplement placé dans une cuillère à café et dissous à l'aide d'un peu d'eau.

Cette solution est donnée à l'enfant en lui demandant de garder le liquide en bouche le plus longtemps possible avant de l'avaler. La posologie d'une gélule par jour est la même que pour les adultes.

Un lot de 420 patients a été traité par une dose quotidienne, par voie sublinguale, de la forme galénique d'oeufs de caille selon l'invention, dans les mêmes indications que les observations précédentes sur une période de 45 jours à 90 jours.

Les résultats cliniques sont surprenants ; ils sont comparables à ceux obtenus avec l'extrait entier, mais sans présenter aucun effet secondaire, a l'exception de 2 % de réactivation de la symptomatologie au cours des huit premiers jours de traitement.

La nouvelle forme galénique selon l'invention, d'utilisation sublinguale, est utilisable chez l'adulte et l'enfant à partir de 5 ans. Par rapport aux oeufs entiers qu'il faut déglutir, malgré les résultats très encourageants qu'ils permettent d'obtenir, l'utilisation sublinguale d'une dilution notamment en 3DH est beaucoup plus simple et les résultats sont excellents sans présenter les inconvénients de l'absorption des oeufs entiers.

Enfin, il faut souligner la grande souplesse d'utilisation et de l'absence d'effets secondaires, du fait qu'il s'agit d'une thérapeutique de terrain non spécifique d'une allergie donnée et que l'éviction de l'allergène est loin d'être indispensable à l'action du produit.

La nouvelle forme galénique comprend des gélules de poudre ou des globules homéopathiques ; elle peut aussi comprendre toutes autres formes telles que comprimés et lyophilisats.

Devant un tel succès clinique (sans désensibilisation, sans corticoïdes, avec le cas échéant un traitement antihistaminique occasionnel), il est logique de préconiser l'emploi, dans le traitement des maladies allergiques, d'un tel produit naturel de nature isothérapique.

On a pu, d'autre part, déterminer qu'un comprimé de 400 mg préparé à partir de la troisième dilution hahnemanienne renferme 1,2 mg de produit actif, ce qui permet l'utilisation en allopathie.

Ces applications concernant les médicaments allopathiques sont spécialement intéressantes pour le traitement des maladies allergiques.

Le traitement des maladies allergiques telles que les rhinites perannuelles, rhinites polliniques, conjonctivite, trachéite spasmodique, asthme, eczéma, peut être donné soit en traitement de fond, soit à titre symptomatique.

La posologie est la même chez l'adulte et chez l'enfant.

On propose comme traitement symptomatique une dose chaque matin à jeun, pendant six semaines à deux mois.

En traitement de fond, on préconise une gélule le matin à jeun pendant six mois à un an.

Les résultats sont excellents et ont été d'abord appréciés en double aveugle contre placebo, puis en ouvert.

L'amélioration se situe toujours entre 70 et 75 % sur la plupart des symptomes.

Les médicaments selon l'invention sont remarquablement bien tolérés du point de vue clinique et aucune anomalie biologique n'a été décelée, avant et après les traitements.

Tous ces éléments en font des médicaments de choix et de première intention dans le traitement de fond et le traitement symptomatique, compte tenu de leur bonne tolérance et de leur efficacité.

## Revendications (Revendications pour l'(les) Etat(s) contractant(s) suivant(s): AT, BE, CH, DE, FR, GB, IT, LI, LU, NL, SE)

1. Composition médicamenteuse à base d'oeufs de caille (coturnix ovum), caractérisée en ce qu'elle comprend un support divisé adapté à un délitement per et sublingual du principe actif, et une partie aliquote d'oeufs de caille obtenue par dilution hahnemanienne d'une solution mère dans de l'eau contenant un mélange en parties pondérales égales de jaunes et de blancs d'oeufs, dont la concentration dosable est au plus égale à 3 DH (troisième dilution hahnemanienne), et contenue sur ledit support.

2. Composition selon la revendication 1, caractérisée en ce que le support est choisi parmi les poudres, les gélules, les globules et les comprimés.

3. Composition selon la revendication 1, caractérisée en ce que les oeufs de caille appartiennent à la souche *B mina*.

## Revendications (Revendications pour l'(les) Etat(s) contractant(s) suivant(s): ES, GR)

1. Procédé d'obtention d'une composition médicamenteuse à base d'oeufs de caille (coturnix ovum), caractérisé en ce qu'on prépare une partie aliquote d'oeufs de caille par dilution hahnemanienne d'une solution mère dans de l'eau contenant un mélange en parties pondérales égales de jaunes et de blancs d'oeufs, dont la concentration dosable est au plus égale à 3 DH (troisième dilution hahnemanienne), et on introduit ladite partie aliquote ainsi préparée dans un support divisé adapté à un délitement per et sublingual du principe actif.

2. Procédé selon la revendication 1, caractérisé en ce que le support est choisi parmi les poudres, les gélules et les comprimés.

3. Procédé selon la revendication 1, caractérisé en ce que les oeufs de caille appartiennent à la souche B mina.

## Claims (Claims for the following Contracting State(s): AT, BE, CH, DE, FR, GB, IT, LI, LU, NL, SE)

1. Medicinal composition based on quail eggs (coturnix ovum), characterized in that it comprises a divided support suited to per- and sublingual disintegration of the active ingredient, and an aliquot portion of quail eggs which is obtained by Hahnemann dilution of a mother solution in water containing a mixture in equal parts by weight of egg yolks and whites, the dosable concentration of which equals not more than 3 HD (third Hahnemann dilution), and is contained on the said support.

2. Composition according to Claim 1, characterized in that the support is selected from powders, capsules, globules and tablets.

3. Composition according to Claim 1, characterized in that the quail eggs belong to the B mina strain.

## Claims (Claims for the following Contracting State(s): ES, GR)

1. Process for obtaining a medicinal composition based on quail eggs (coturnix ovum), characterized in that an aliquot portion of quail eggs is prepared by Hahnemann dilution of a mother solution in water containing a mixture in equal parts by weight of egg yolks and whites, the dosable concentration of which equals not more than 3 HD (third Hahnemann dilution), and the said aliquot portion thus prepared is introduced into a divided support suited to per- and sublingual disintegration of the active ingredient.

2. Process according to Claim 1, characterized in that the support is selected from powders, capsules and tablets.

3. Process according to Claim 1, characterized in that the quail eggs belong to the B mina strain.

## Patentansprüche (Patentansprüche für folgende(n) Vertragsstaat(en): AT, BE, CH, DE, FR, GB, IT, LI, LU, NL, SE)

1. Medikamentöse Zusammensetzung auf der Basis von Wachteleiern (coturnix ovum), dadurch gekennzeichnet, daß diese einen teiligen Träger, der für eine perlinguale und eine sublinguale Auflösung des Wirkstoffes geeignet ist und einen aliquoten Teil an Wachteleiern aufweist, der durch ein Hahnemann'sches Verdünnen einer Urtiterlösung mit Wasser erhalten wurde, die eine Mischung von gleichen Gewichtsanteilen an Eigelb und an Eiweiß enthält, wobei dessen bestimmbare Konzentration höchstens gleich D 3 (dritte Hahnemann'sche Verdünnung) ist, und der auf dem Träger aufgenommen ist.

2. Zusammensetzung nach Anspruch 1, dadurch gekennzeichnet, daß der Träger ausgewählt ist aus Pulvern, Gelatinekapseln, Globuli und Tabletten.

3. Zusammensetzung nach Anspruch 1, dadurch gekennzeichnet, daß die Wachteleier dem Stamm B mina zugehörig sind.

## Patentansprüche (Patentansprüche für folgende(n) Vertragsstaat(en): ES, GR)

1. Verfahren zum Herstellen einer medikamentösen Zusammensetzung auf der Basis von Wachteleiern (coturnix ovum), dadurch gekennzeichnet, daß man einen aliquoten Teil an Wachteleiern durch Hahnemann'sches Verdünnen einer Urtiterlösung mit Wasser herstellt, die eine Mischung von gleichen Gewichtsanteilen an Eigelb und Eiweiß enthält, wobei die bestimmbare Konzentration des Teiles höchstens gleich D 3 (dritte Hahnemann'sche Verdünnung) ist, und man führt den so hergestellten aliquoten Teil in einen teiligen Träger ein, der für eine perlinguale und eine sublinguale Auflösung des Wirkstoffes geeignet ist.

2. Verfahren nach Anspruch 1, dadurch gekennzeichnet, daß der Träger ausgewählt ist aus Pulvern, Gelatinekapseln, Globuli und Tabletten.

3. Verfahren nach Anspruch 1, dadurch gekennzeichnet, daß die Wachteleier dem Stamm B mina zugehörig sind.
